(19) Europäisches Patentamt / European Patent Office / Office européen des brevets

(11) **EP 3 235 560 A1**

(12) **EUROPÄISCHE PATENTANMELDUNG**

(43) Veröffentlichungstag:
25.10.2017  Patentblatt 2017/43

(51) Int Cl.:
*B01J 8/22* (2006.01)    *B01J 8/00* (2006.01)
*C07C 69/54* (2006.01)   *C07C 67/39* (2006.01)

(21) Anmeldenummer: 16166606.0

(22) Anmeldetag: 22.04.2016

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**MA MD**

(71) Anmelder: **Evonik Röhm GmbH**
**64293 Darmstadt (DE)**

(72) Erfinder:
• **Lygin, Alexander**
  **64347 Griesheim (DE)**
• **Grömping, Matthias**
  **64295 Darmstadt (DE)**
• **Krill, Steffen**
  **64367 Mühltal (DE)**

(54) **VERFAHREN ZUR DURCHFÜHRUNG EINER HETEROGEN-KATALYSIERTEN REAKTION**

(57)  Die vorliegende Erfindung betrifft ein neuartiges Verfahren zur Durchführung einer heterogenkatalysierten Reaktion, insbesondere in einer Flüssigphase.

Heterogenkatalysierte Reaktionen in einer flüssigen Phase sind in der Fachliteratur ausgiebig beschrieben. Dazu gehören z.B. die Cobalt-katalysierte Fischer-Tropsch Synthese, Palladium- und Nickel-katalysierte direkte Hydrierungen mit Wasserstoff, zahlreiche Oxidationsreaktionen.

Vor diesem Hintergrund ist es mit dem vorliegenden erfindungsgemäßen Verfahren gelungen, solche Verfahren bei gleichbleibenden oder sogar gesteigerten Aktivitäten und Selektivitäten länger störungsfrei zu betreiben. Hieraus ergibt sich die Möglichkeit, solche Verfahren möglichst einfach, wirtschaftlich und umweltschonend durchzuführen.

EP 3 235 560 A1

**Beschreibung**

**Gebiet der Erfindung**

Die vorliegende Erfindung betrifft ein neuartiges Verfahren zur Durchführung einer heterogen-katalysierten Reaktion, insbesondere in einer Flüssigphase.

Heterogenkatalysierte Reaktionen in einer flüssigen Phase sind in der Fachliteratur ausgiebig beschrieben. Dazu gehören z.B. die Cobalt-katalysierte Fischer-Tropsch Synthese, Palladium- und Nickel-katalysierte Hydrierungen mit Wasserstoff, zahlreiche Oxidationsreaktionen.

Vor diesem Hintergrund ist es mit dem vorliegenden erfindungsgemäßen Verfahren gelungen, solche Verfahren bei gleichbleibenden oder sogar gesteigerten Aktivitäten und Selektivitäten länger störungsfrei zu betreiben. Hieraus ergibt sich die Möglichkeit, solche Verfahren möglichst einfach, wirtschaftlich und umweltschonend durchzuführen.

**Stand der Technik**

Heterogen-katalysierte Verfahren, bei denen zumindest eine flüssige Phase vorliegt, werden häufig in so genannten Slurry-Reaktoren durchgeführt. Slurry-Reaktoren finden ihre Anwendung vor allem für heterogen-katalysierte Prozesse, für die eine gute Durchmischung und geringe Temperatur- und Konzentrationsgradienten von Vorteil sind. Besonders für stark exotherme Reaktionen ist es wichtig die Reaktionswärme so gut wie möglich abzuführen. Dafür sind die Reaktoren insbesondere mit interner oder externer Zirkulation des Reaktionsgemisches besonders gut geeignet.

Eine bekannte Variation von solchen Reaktoren sind Reaktoren mit einem Innenrohr (draft tube), die eine interne Zirkulation ermöglichen. So ist in US 5,288,673 ein Einsatz eines Slurry-Reaktors mit Innenrohr für eine Fischer-Tropsch Synthese zur Herstellung von Kohlenwasserstoffen aus Synthesegas beschrieben.

In CN 104418309 ist ein Slurry-Reaktor mit Innenrohr für die Wasserstoffperoxidproduktion in einer heterogen-katalysierten Hydrierungsreaktion von Anthraquinon beschrieben. Die eingesetzte Katalysatorkonzentration liegt bei ca. 10 g/L (< 0,01 kg/kg Gemisch) und ist somit relativ gering. Die Strömungsrichtung im Innenrohr von unten nach oben fördert einen großen Anteil vom Katalysator immer wieder in das Rohr zurück.

In WO 2012/152600 ist eine Ammoximation von Cyclohexanon beschrieben, die mit einem heterogenen TS-1 Katalysator als eine Dreiphasenreaktion (gasförmig-flüssig-fest) durchgeführt wird. Sowohl der Wärmeübergang als auch der Stoffübergang in diesem Prozess können deutlich verbessert werden, wenn ein zylindrisches Innenrohr zum Einsatz kommt. Dabei werden die Edukte an unterschiedlichen Stellen zudosiert. Eine Zudosierung erfolgt unter dem Innenrohr (hier $NH_3$), eine von oben (hier $H_2O$) und optional eine seitlich (hier beispielsweise Cyclohexanon). Die Filtration erfolgt mithilfe von vielen Kerzenfilter mit einer großen Gesamtfläche. Diese sind mittig in der Reaktorhöhe bzw. an dem äußeren Rande des Innenrohrs platziert. Der Prozess lässt sich anhand der Beschreibung für 1 Jahr ohne Unterbrechungen und Filterrückspülung betreiben. Nach einem Jahr müssen dann die Filter gereinigt werden.

Der Einsatz von Slurry-Reaktoren mit interner Zirkulation für die Reaktionen, in der eine Bildung von Ablagerungen möglich ist, wie insbesondere für Reaktionen, bei denen z.B. polymerisierbare Substanzen erzeugt werden, ist nicht beschrieben. In der US 5,417,930 wird sogar angedeutet, dass ein Slurry-Typ Reaktor mit interner Zirkulation über ein oder mehrere Innenrohre für die Polymerisation von polymerisierbaren Stoffen besonders förderlich sein kann.

Für Reaktionen, die solche Substanzen aufweisen, finden sich daher im Stand der Technik auch einige Reaktoren für die Durchführung einer heterogen-katalysierten Reaktion mit einer externen Zirkulation des Slurry-Gemisches. So wird beispielsweise in der US 5,969,178 ein Verfahren zur kontinuierlichen Herstellung von MMA aus Isobuten oder tert-Butanol über Methacrolein beschrieben Dabei findet eine oxidative Veresterung von leicht polymerisierbarem Methacrolein als letzter Schritt des Verfahrens in einer Blasensäule mit externer Zirkulation statt. Der Reaktor wird dazu als ein "External circulation type bubble column reactor" beschrieben.

Die CN 101314120 beschreibt einen Loop-Slurry-Reaktor mit externer Zirkulation des Slurrygemisches für Durchführung von z.B. Fischer-Tropsch Prozess.

Alle Reaktoren mit einer externen Zirkulation des Slurrygemisches erfordern ziemlich aufwendige Reaktorausführungen und Slurry-Fördervorrichtungen, die beispielsweise mittels weiterer Pumpen sichergestellt werden müssen. Deswegen und aus weiteren Gründen weisen diese Systeme somit Nachteile gegenüber Systemen mit interner Zirkulation auf.

Zusammengefasst sind die folgenden Aspekte des Verfahrens gemäß Stand der Technik verbesserungswürdig und wünschenswert:

- Möglichst einfaches Aufbauprinzip des Reaktors und damit verbunden eine unbeschränkte Eignung für eine Hochskalierung

- Der Einsatz von sich absetzenden oder leicht polymerisierbaren Stoffen ist möglich

- Einsatz von hohen Katalysatorkonzentrationen und damit ein höherer Durchsatz

- Eine verbesserte Abriebfestigkeit des eingesetzten heterogenen Katalysators

- Gute Durchmischung der Reaktorphasen

- Lange Standzeit des Katalysators, robuster Betrieb ohne Unterbrechungen, keine oder sehr kurze Wartungsphasen

- Die Möglichkeit der Installation vereinfachter Filtrationssysteme für eine kontinuierliche Abtrennung vom heterogenen Katalysator aus dem Slurrygemisch ohne Shutdown-Zeiten

**Aufgabe**

In Anbetracht des Standes der Technik ist es daher Aufgabe der vorliegenden Erfindung, ein technisch verbessertes Verfahren zur Durchführung einer heterogen-katalysierten Reaktion, insbesondere in einer Flüssigphase, zur Verfügung zu stellen. Dieses neuartige Verfahren soll insbesondere mit weniger Nachteilen als herkömmliche Verfahren des Standes der Technik behaftet sein.

Insbesondere sollen Verfahren des Standes der Technik dergestalt verbessert werden, dass nur ein minimaler Katalysatorabrieb erfolgt und dadurch eine lange Standzeit des eingesetzten heterogenen Katalysators bei gleichzeitig guter und nahezu konstanten Katalysatoraktivität, Selektivität und guter Durchmischung im Reaktor möglich ist.

Darüber hinaus soll das Verfahren beim Einsatz von leicht polymerisierbaren Edukten und Bildung solcher Produkten und/oder Nebenprodukten eine solche Reaktorausführung ermöglichen, dass diese nur eine maximal sehr geringe Polymerisation zulässt.

Weiterhin soll das Verfahren gegenüber dem Stand der Technik kostengünstig sein, insbesondere ohne größere Katalysatorverluste durch Abrieb oder Austrag durchführbar sein und mit weniger und kürzeren Unterbrechungen des Betriebes erfolgen können.

Ferner sollte das Verfahren mit relativ einfachen und kostengünstigen Anlagen durchgeführt werden können. Die Anlagen sollten demgemäß mit geringen Investitionskosten verbunden sein. Hierbei sollen die Anlagen einfach zu warten sein, geringen Unterhaltskosten verursachen und sicher zu betreiben sein.

Weitere nicht explizit genannte Aufgaben ergeben sich aus dem Gesamtzusammenhang der nachfolgenden Beschreibung und der Ansprüche.

**Lösung**

Gelöst werden diese Aufgaben durch die Zurverfügungstellung eines neuartigen Verfahrens zur Durchführung einer heterogen-katalysierten Reaktion in einem Dreiphasen-Reaktor. Dieses neuartige Verfahren ist dadurch gekennzeichnet, dass sich in dem Reaktor mindestens eine flüssige, mindestens eine gasförmige und mindestens eine feste Phase befinden. Dabei weist der Reaktor mindestens zwei Zonen auf. In Zone 1 wird das Reaktionsgemisch nach unten gefördert In Zone 2 wird das Reaktionsgemisch wiederum nach oben gefördert. Dabei sind die Zonen 1 und 2 durch eine Trennwand voneinander getrennt. Während des Reaktorbetriebes bleibt in Zone 1 in der Regel wesentlich weniger Katalysatormasse pro Volumeneinheit schwebend als in Zone 2. So ist das Verhältnis zwischen den durchschnittlichen Katalysatorkonzentrationen in Zone 2 und in Zone 1 größer als 2, bevorzugt größer 5, insbesondere größer 10 und besonders bevorzugt größer 20. In ganz besonders bevorzugten Ausführungsformen der vorliegenden Erfindung ist das Verhältnis zwischen den durchschnittlichen Katalysatorkonzentrationen in Zone 2 und in Zone 1 sogar größer als 100.

Zone 1 weist optimalerweise eine turbulente Strömung und dadurch eine sehr schnelle Durchmischung auf, während Zone 2 zumindest im oberen Teil eine laminare Strömung aufweist, die für eine optimale Katalysatorabsetzung förderlich ist.

Bevorzugt ist eine Ausführung des erfindungsgemäßen Verfahrens, bei der das für den Prozess benötigte Gas sich im laufenden Betrieb fast ausschließlich in Zone 2 befindet, während Zone 1 vom ungelösten Gas weitestgehend befreit bleibt.

Ein Merkmal einer bevorzugten Ausführung der vorliegenden Erfindung ist die Existenz eines Konzentrationsgradienten entlang der Reaktorhöhe in Zone 2: der größte Anteil der kompletten Katalysatormasse befindet sich im unteren Teil der Zone 2, während nur ein Bruchteil davon sich im oberen Teil der Zone 2 befindet. Daraus ergibt sich ein Verhältnis zwischen der Katalysatorkonzentration in Zone 2 auf 90% der von unten gemessenen Füllhöhe des Reaktors zu der Katalysatorkonzentration auf 20% der von unten gemessenen Füllhöhe kleiner 0,3. Besonders bevorzugt ist dieses Verhältnis kleiner 0,2, noch bevorzugter kleiner 0,1 und ganz besonders bevorzugt kleiner 0,05. Während sich somit in der Zone 2 erfindungsgemäß ein Katalysatorkonzentrationsprofil bildet, bleibt die Katalysatorkonzentration in der Zone 1 mehr oder weniger konstant auf dem Niveau der minimalen Katalysatorkonzentration im oberen Teil der Zone 2. Somit, beispielsweise in einer Ausführungsform mit einem Rührer in Zone 1, kommt der Rührer nur mit einem Bruchteil der Gesamtkatalysatormenge in Kontakt. Das resultiert in einem deutlich geringeren Katalysatorabrieb und erhöhter Standzeit des Katalysators. Somit werden auch die in der Regel installierten Filter, die für die Filtration des Produktgemisches eingesetzt sind, geschont und brauchen weniger bis keine Rückspülung und/oder keinen Ersatz.

Diese Verhältnisse können erfindungsgemäß insbesondere durch eine optimale Durchführung der Reaktion in einem erfindungsgemäßen Reaktor realisiert werden. Wie bereits beschrieben kann eine solche optimale Durchführung beispielsweise dadurch gekennzeichnet sein, dass in der Zone 2 entlang der Reaktorhöhe sich ein Katalysatorkonzentrationsgradient bildet, wobei die maximale Katalysatorkonzentration sich nahe des Reaktorbodens befindet, während sich die minimale Katalysatorkonzentration im oberen Teil des Reaktors befindet. Eine solche Katalysatorkonzentrationsverteilung kann wiederum durch einen optimierten Strömungsverlauf innerhalb des Reaktors erzeugt werden. Dazu können die im Weiteren erfolgenden Ausführungen angewendet werden.

Weiterhin bevorzugt oder ergänzend zu einer oder beiden der vorgenannten bevorzugten Ausführungsformen der Erfindung ist Verfahren bei dem das Verhältnis zwischen der durchschnittlichen vertikalen Strömungsgeschwindigkeit in der Zone 1 und der durchschnittlichen vertikalen Strömungsgeschwindigkeit in der Zone 2 zwischen 2 und 100, besonders bevorzugt zwischen 5 und 50 und insbesondere bevorzugt zwischen 10 und 40 beträgt.

In einer besonders bevorzugten Ausführungsform der vorliegenden Erfindung wird die interne Zirkulation zwischen den Zonen 1 und 2 im Reaktor - unabhängig von den übrigen Einstellungen des Verfahrens - mittels einem Innenrohr (7) gewährleistet. Dabei wird der Strom des Reaktionsgemischs im Innenrohr (Zone 1) mit Fließrichtung nach unten erzeugt, während in der außenliegenden Zone 2 ein Gegenstrom - von unten nach oben erzeugt wird. Bei dieser Zone 2 handelt es sich dann um den Bereich zwischen dem Innenrohr und den Reaktorwänden.

Bei einer solchen "geodätischen" Fahrweise sind die Abwärtsbewegung des Reaktionsgemisches in der Zone 1 und die Aufwärtsbewegung in der Zone 2 optimal aufeinander abgestimmt.

Zone 1 ist bevorzugt ein zylindrisches Innenrohr, wobei der Durchmesser dieses Innenrohrs entlang der Reaktorhöhe variiert werden kann. So ist z.B. bevorzugt, dass der untere Teil des Rohres einen geringeren Durchmesser als der obere Teil dieses Rohres aufweist. So ist eine möglichst schnelle Strömungsgeschwindigkeit unten und eine wesentlich geringere oben gewährleistet, was die Menge vom ins Rohr wieder gelangten und vorher nicht abgesetzten Katalysator überraschend deutlich minimiert.

Das kann damit erklärt werden, dass eine solche Verbreitung der Zone 1 im oberen Teil einen fließenden Übergang zwischen der turbulenten Zone 1 und laminaren Zone 2 erleichtert und dadurch weniger Katalysator in Zone 1 mitgerissen wird.

Dies hat weiterhin den überraschenden Vorteil, dass durch beispielsweise ein Rühren oder Pumpen im Rohr ein geringer Katalysatorabrieb auftritt. Das bevorzugte Verhältnis zwischen dem Durchmesser des Rohrs im oberen und unteren Teil der Zone 1 liegt zwischen 1 und 5, bevorzugt zwischen 2 und 4.

Der Reaktor hat bevorzugt eine typische für Druckreaktoren zylindrische Form, abgerundet im unteren und oberen Teil. Das optimale Verhältnis zwischen der Reaktorhöhe und dem Reaktordurchmesser liegt bevorzugt zwischen 1 und 3, besonders bevorzugt zwischen 1,1 und 2,5, ganz besonders bevorzugt zwischen 1,3 und 2,3.

Der Durchmesser des Reaktors kann entlang der Reaktorhöhe variiert werden. So ist z.B. bevorzugt, dass der untere Teil des Reaktors einen geringeren Durchmesser als der obere Teil dieses Reaktors aufweist. So kann gewährleisten werden, dass im unteren Teil des Reaktors eine optimale Durchmischung des Reaktionsgemisches mit dem Katalysator, während im oberen Reaktorteil eine möglichst langsame Strömungsgeschwindigkeit nach oben und gute Sedimentation des Katalysators erzeugt wird. So kann deutlich weniger nicht abgesetzten Katalysators aus der Zone 2 in die Zone 1 wieder gelangen und somit ein deutlich geringerer Katalysatorabrieb durch den Rührer in der Zone 1 erfolgen.

Unabhängig von der weiteren Durchführung des Verfahrens ist ein weiteres einstellbares Merkmal der Erfindung das Verhältnis des Reaktordurchmessers im oberen Teil zu dem im unteren Teil, das bevorzugt zwischen 1 und 2, bevorzugt zwischen 1,1 und 1,5 liegt. Besonders bevorzugt liegt das Verhältnis zwischen dem maximalen und dem minimalen Reaktordurchmesser zwischen 1 und 2, besonders bevorzugt zwischen 1,1 und 1,5.

Zur genauen Bestimmung der jeweiligen Verhältnisse ist die Formulierung "im oberen Teil" zum Beispiel derart zu bestimmen, dass eine Messung 10% der Gesamthöhe unterhalb des obersten Endes des jeweiligen Bereichs gemessen wird. Analog bedeutet die Formulierung "im unteren Teil", dass dieser Bereich entsprechend 10% oberhalb des unteren Endes der Gesamthöhe als Messpunkt gewählt wird. Wichtig ist zur genaueren Bestimmung, dass der Abstand zum oberen bzw. zum unteren Ende jeweils identisch ist, und dass dieser Abstand zur Messung maximal eine Strecke entsprechend 20% der Gesamthöhe der zu vermessenden Vorrichtung (beispielsweise Zone 1 oder Gesamtreaktor) vom jeweiligen oberen und unteren Ende der Vorrichtung entfernt ist.

Überraschenderweise hat sich gezeigt, dass eine solche Reaktorform mit einem Innenrohr und interner Zirkulation, wie oben beschrieben, es nicht nur ermöglicht, einen reibungslosen Betrieb, hohe Katalysatoreffektivität, eine gute Durchmischung und einen guten Wärmeabfuhr zu erhalten, sondern auch den eingesetzten heterogenen Katalysator in einem solchen Reaktor derart schont, dass der Katalysatorabrieb minimiert werden kann und die Katalysatorstandzeit insgesamt erhöht wird. Dadurch können zusätzlich auch die eingesetzten Filter störungsfrei für längere Zeit betrieben werden ohne dass eine aufwändige Reinigung und/oder Filterersatz nötig wäre.

So ist es besonders vorteilhaft den Prozess so zu gestalten, dass im unteren Teil des Reaktors eine optimale Durchmischung des Reaktionsgemisches mit dem Katalysator, während im oberen Reaktorteil eine möglichst langsame Strömungsgeschwindigkeit nach oben und gute Sedimentation des Katalysators bewirkt werden. So kann deutlich weniger nicht abgesetzten Katalysators aus der Zone 2 in die Zone 1 gelangen und somit ein deutlich geringerer Katalysatorabrieb durch den Rührer in der Zone 1 stattfinden.

Weitere überraschende Vorteile des vorliegenden Verfahrens gegenüber dem Stand der Technik ist unter anderem, dass gute Wärme- und Stoffübergänge erfolgen und dadurch nur sehr geringe Temperatur- und Konzentrationsgradienten entstehen was für eine hohe Aktivität und Selektivität des Katalysators sehr förderlich ist. Weiterhin liegt eine für die Dreiphasenreaktion optimale Gasverteilung im Reaktor vor. Darüber hinaus ist eine sehr robuste Durchführung einer heterogen-katalysierten Reaktion mit hohen Katalysatorkonzentrationen durch das erfindungsgemäße Verfahren möglich.

Insbesondere überraschend hat sich gezeigt, dass der spezifische Reaktoraufbau, wie beispielsweise bestimmte Verhältnisse der Reaktorabmessungen, der Einbau eines Innenrohrs zum Katalysatorschutz, Position und Aufbau des Sedimentations- und Filtersystems eine große Rolle für einen stabilen und effizienten Betrieb mit relativ hohen Katalysatorkonzentrationen ohne nennenswerten Katalysatorabrieb bewirken.

Unabhängig von den anderen gewählten Ausführungsformen der Erfindung wird das Reaktionsgemisch in der Zone 1 bevorzugt mittels mindestens einer Pumpe oder mindestens eines Rührers nach unten gefördert. Alle Rührer, die eine axiale Strömung nach unten fördern würden hierzu besonders passen. Bevorzugt wird mindestens ein besonders bevorzugt mindestens zwei Propellerrührer eingesetzt. Im Falle eines Rührers ist dieser bevorzugt ca. in der Mitte des Innenrohrs platziert, während zwei Rührer bevorzugt in der Mitte und am unteren Rande des Innenrohrs eingebaut werden.

Weiterhin unabhängig von den anderen gewählten Ausführungsformen der Erfindung wird bevorzugt mindestens ein flüssiger Feedstrom in den oberen Teil der Zone 1 eingeleitet. Besonders bevorzugt werden alle flüssigen Feedströme in den oberen Teil der Zone 1 eingeleitet.

Da der Reaktor bevorzugt für einen kontinuierlichen Prozess eingesetzt werden soll, sollte bevorzugt der heterogene Katalysator vom Reaktionsgemisch kontinuierlich abfiltriert werden. Dazu werden bevorzugt sich im Reaktor, besonders bevorzugt an der Peripherie im oberen Teil der Zone 2 des Reaktors, befindende Filter eingesetzt. Insbesondere wird unabhängig von den anderen gewählten Ausführungsformen der Erfindung bevorzugt im oberen Teil der Zone 2 mindestens ein kontinuierlich betreibbarer und zurückspülbarer Filter installiert.

Alternativ oder zusätzlich dazu und gleichsam bevorzugt wird das Reaktionsgemisch kontinuierlich aus dem Reaktor ausgetragen und über mindestens einen externen Filter filtriert. Danach wird der Katalysator nach der Filtration optional weiterbehandelt und teilweise oder vollständig zurück in den Reaktor geleitet. Bei dieser Weiterbehandlung kann es sich z.B. um ein Waschen, Reaktivieren oder eine Trennung nach Partikelgrößen handeln.

Vor solche Filter kann bevorzugt, beispielsweise auch an der Peripherie des Reaktors, ein zusätzliches Absetzungssystem installiert werden. Dabei kann es sich um eine spezielle Zone mit laminarer Strömung, wo Sedimentation eines Großteils des eingesetzten Katalysators erfolgt, handeln. Eine solche Sedimentation erfolgt damit bevor es zur tatsächlichen Filtration kommt. Eine mögliche Variante eines solchen Sedimentationssystems ist z.B. ein Komplex aus geneigten Elementen wie z.B. Röhren oder geneigten Metallblechen (z.B. ein Schrägklärer). Das Funktionsprinzip solcher Systeme ist weiter in Journal of Fluid Mechanics / Volume 92 / Issue 03 / June 1979, pp 435- 457 und "Enhanced sedimentation in vessels having inclined walls" in Theory of Dispersed Multiphase Flow: Proceedings of an Advanced Seminar Conducted by the Mathematics Research Center The University of Wisconsin-Madison May 26-28, 1982 beschrieben. Die Anwendung für eine z.B. Oxidationsreaktion ist in JP 10-094705 A und JP 09-248403 A nachzulesen.

Besonders bevorzugt, befindet sich das Sedimentationssystem und Filter im oberen Teil des Reaktors an einer derartigen Position der Zone 2, dass die Strömungsgeschwindigkeit dort am langsamsten ist. Das bedeutet wiederum, dass der Querschnitt der Zone 2 an dieser Stelle eine maximale Fläche aufweist.

Die bevorzugt eingesetzte Filterporosität beträgt zwischen 5 und 100 Mikrometer, besonders bevorzugt zwischen 10 und 50 Mikrometer. Für einen zusätzlichen Rückhalt der feinen Katalysatorpartikel wird das einmal über Reaktorfilter (5) filtrierte Reaktionsgemisch bevorzugt mindestens noch einmal über feinere Filter mit der Porosität von 1 bis 10 $\mu$m außerhalb vom Reaktor gefiltert, so dass die Partikel von maximal 5 $\mu$m zu mindestens 90% von dem Filter zurückgehalten werden.

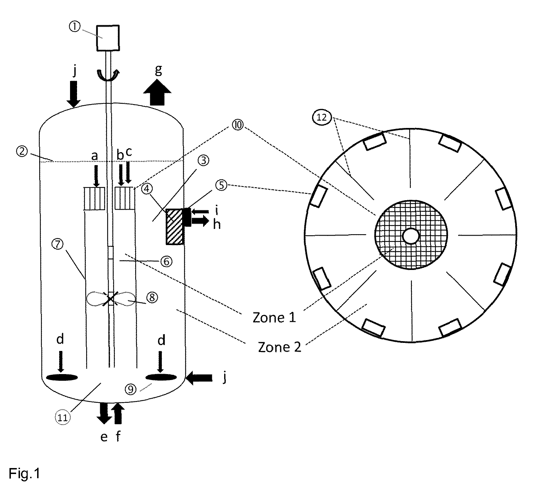

In einer beispielhaften und besonders bevorzugten Ausführungsform gemäß Fig. 1 passiert das Reaktionsgemisch nach dem Sedimentationssystem (4) mehrere rund um den Reaktor gleichmäßig verteilte Filter (5) und gelangt zu den weiteren Produktaufarbeitungsschritten. Das Filter- und Sedimentationssystem wird bevorzugt regelmäßig zurückgespült, damit eine maximale Menge des heterogenen Katalysators im Reaktor katalytisch aktiv bleibt und keine Verstopfung des Sedimentationssystems und der Filter erfolgt.

Unabhängig von einer Filtration können sich bevorzugt im oberen Teil der Zone 1 ein oder mehrere Strömungsbrecher (10), so genannte Drallbrecher, befinden. Diese wirken der Wirkung eines Vortex, bzw. Trichters, entgegen, indem die Aufwirbelung der Flüssigkeit gebrochen wird und ein ruhiger Übergang zwischen dem Bereich außerhalb und innerhalb der Zone 1, bzw. des Innenrohrs ermöglicht wird. Besonders bevorzugt wird die Zone 2 mit wenigstens 2, besonders bevorzugt mit wenigstens 4, ganz besonders bevorzugt mit wenigstens 8 Strömungsbrecher und/oder Trennwänden ausgestattet. Somit kann die radiale Strömungsgeschwindigkeit in Zone 2 drastisch reduziert werden und die Sedimentation im oberen Teil der Zone 2 besonders effektiv gestaltet werden.

Bevorzugt wird das für die Reaktion benötigte Gas über die Gasverteiler (9), so genannte Sparger, im unteren Reaktorteil im feindispergiertem Zustand eindosiert. Bevorzugt wird das eingesetzte Gas in die Richtung zum Reaktorboden eindosiert, damit möglichst weniger Verstopfung mit den Katalysatorpartikeln auftreten kann.

Im Falle einer Hydrierungsreaktion wird zweckgemäß Wasserstoff oder Wasserstoff-haltiges Gas verwendet. Im Falle von Oxidationsreaktion wird Sauerstoff in Form von Luft oder eines anderen $O_2$-haltigen Gemisches benutzt. Im Falle von Fischer-Tropsch Synthese kann Synthesegas als Gas dienen. Auch andere nicht genannte Gase können je nach gewünschter Reaktion verwendet werden.

Eine für den beschriebenen Reaktor passende Flüssigphasenanwendung ist z.B. die Hydrierung von Anthraquinon, die für die Wasserstoffperoxidproduktion verwendet wird. Außerdem kann damit eine Fetthärtung, also Hydrierung von ungesättigten Fettsäuren betrieben werde. Zahlreiche andere Hydrierungen z.B. von Mehrfachbindungen aufweisenden Stoffen, wie Aromaten, Alkenen oder Alkinen, Nitroverbindungen, Carbonylverbindungen etc. lassen sich auch mit dem Reaktortyp durchführen.

Besonders bevorzugt ist das erfindungsgemäße Verfahren bei einer heterogen-katalysierten Oxidationsreaktion mit einem Sauerstoff-haltigen Gas anwendbar. Dabei weist die Sauerstoffkonzentration ($O_2$-Partialdruck) in Zone 2 einen Gradient auf mit einer maximalen $O_2$-Konzentration im unteren Teil der Zone 2 und einer minimalen $O_2$-Konzentration im oberen Teil dieser Zone. Besonders bevorzugt ist in einem solchen Verfahren das Verhältnis zwischen der Sauerstoffkonzentration in der Gasphase der Zone 2 auf 20% der von unten gemessenen Füllhöhe des Reaktors und der Sauerstoffkonzentration in der Gasphase der Zone 2 auf 90% der von unten gemessenen Füllhöhe größer als 2, bevorzugt größer als 4.

Einige Beispiele für die passenden Oxidationsprozesse in der flüssigen Phase sind z.B. spezielle Oxidationen von Alkenen, Alkylaromaten, Oxidative Veresterung von Aldehyden zu Carbonsäureester, wie z.B. die Umsetzung von (Meth)acrolein zu Alkyl (Meth)arylat, und weitere selektive Oxidationsreaktion im Bereich der Spezialchemie.

Ganz besonders bevorzugt handelt es sich bei der heterogen-katalysierten Reaktion um eine kontinuierliche oxidative Veresterung von Methacrolein mit Sauerstoff und Methanol zur Herstellung von Methylmethacrylat.

Die eingesetzten heterogenen Katalysatoren sind bevorzugt edelmetallhaltige, insbesondere Pt-, Pd-, Ru-, Rh-, Ru-, Au- und/oder Ag-haltige, geträgerte Katalysatoren. Als Träger können insbesondere mineralische Oxide, Oxidgemische, Aktivkohle, Polymermaterialen oder andere Stoffe eingesetzt werden. Weiterhin hat ein für eine solche Oxidationsreaktion bevorzugt eingesetzte Katalysator einen mittleren Durchmesser zwischen 10 und 200 $\mu$m.

Der eingesetzte Katalysator kann z.B. für einen Wasch- und/oder Regeneriervorgang, für kontinuierliche Monitoring/Analyse oder Erneuerung dem Reaktor kontinuierlich oder diskontinuierlich entnommen werden. Die Anschlussstelle für Katalysatorentnahme oder Zufuhr befinden sich bevorzugt im unteren Teil des Reaktors, wo die Katalysatorkonzentration am höchsten ist. Eine alternativ bevorzugte Variante impliziert eine Katalysatorentnahmestelle im oberen Teil des Reaktors. In diesem Fall ist eine solche Stelle bevorzugt, an der sich insbesondere die kleinsten Katalysatorpartikel befinden.

Außer den für die Reaktion benötigten Edukten können verschiedene Hilfsstoffe wie z.B. Säuren, Basen, Polymerisationsinhibitoren, Antischaumbildner usw. dem Prozess zugeführt werden.

Alle hochreaktiven (z.B. leicht polymerisierbare) Edukte und/oder Hilfsstoffe, wie z.B. eine starke Base, wie NaOH oder KOH, oder eine starke Säure, wie $H_2SO_4$ oder HCl, sind bevorzugt in den oberen Teil der Zone 1 einzudosieren. Damit wird gewährleistet, dass diese Stoffe möglichst schnell mit dem Reaktionsgemisch durchmischt werden, bevor sie mit dem Katalysator und den anderen Edukten in Kontakt kommen können. Das vermeidet eine lokale Überhitzung und verbessert die Selektivität und Gesamteffektivität der Zielreaktion.

**Zeichnungen**

Bezugszeichenliste Fig. 1

Bei Fig. 1 handelt es sich um die spezifische Ausgestaltung eines im erfindungsgemäßen Verfahren einsetzbaren Reaktors. Dieser stellt dabei eine insbesondere für Oxidationsreaktionen besonders geeignete Ausführungsform der Erfindung dar. Andererseits dient die Zeichnung jedoch nicht dazu, den Schutzumfang der vorliegenden Anmeldung in irgendeiner Weise einzuschränken. Dabei ist diese Zeichnung derart vereinfacht, dass Verjüngungen des Reaktors oder der Zone 1 beispielsweise nicht abgebildet sind.

| | |
|---|---|
| 1: Motor | a: Feed 1 (Edukt 1) |
| 2: Reaktionsgemischspiegel | b: (optional) Feed 2 (Edukt 2) |
| 3: Sedimentationszone (Zone 2) | c: (optional) Feed 3 (Hilfstoff 1) |
| 4: (optional) Sedimentationssystem | d: Gas |
| 5: Filtersystem (mit Rückspülung) | e: Katalysator Slurry Austrag |
| 6: Vermischungs-/Sättigungszone (Zone 1) | f: Katalysator Slurry Eintrag |
| 7: Innenrohr | g: Abgasaustrag (zum Kühler) |
| 8: mindestens ein Propellerrührer | h: Produktgemischaustrag |
| 9: Luftverteilungsdüsen (Sparger) | i: Filterrückspülung |
| 10: Strömungsbrecher (Drallbrecher) | j: (optional) Inertgas-Purge |
| 11: Reaktionszone (Zone 2, unterer Teil) | |
| 12: Segmentblechteile (Strömungsbrecher, Trennwände) | |

**Beispiele**

Beispiel 1

Der Reaktor (gemäß Fig.) hatte folgende Verhältnisse der Abmessungen:

Reaktorhöhe/Reaktordurchmesser = 1,6

Füllhöhe mit Reaktionsgemisch/Reaktorhöhe = 0,75

Reaktordurchmesser (D2) / Innenrohrdurchmesser (D1) = 5,3

Das Verhältnis zwischen der durchschnittlichen vertikalen Strömungsgeschwindigkeiten (nach unten) V1 im Innenrohr (Zone 1) und der der durchschnittlichen vertikalen Strömungsgeschwindigkeiten (nach oben) V2 in der Zone 2 ist: $V1/V2 = (D2/D1)^2-1 = 27,4$

<u>Durchführung einer oxidativen Veresterungsreaktion von Methacrolein zu Methylmethacrylat.</u>

Der pH-Wert einer 42,5 gew%igen Lösung von Methacrolein (MAL) in Methanol wurde durch die Zugabe von einer 1 gew%igen Lösung NaOH in Methanol bei Rühren auf pH = 7 eingestellt. Diese Lösung wurde mit einer konstanten Zugaberate kontinuierlich in den oberen Teil des Innenrohrs (Zone 1) des erfindungsgemäß einsetzbaren Reaktors gemäß Fig. 1 unter 5 bara Druck und 80 °C Innentemperatur zugeführt. Gleichzeitig wurde in diesen Reaktor zusammen mit 600 g Pulverkalalysator $Au/NiO/SiO_2-Al_2O_3-MgO$ (hergestellt nach dem Beispiel 1 aus der Anmeldung EP 2 210 664 A1) so viel 1 gew%ige NaOH-Lösung in Methanol zugeführt (auch in den oberen Teil des Innenrohrs), dass der Wert pH = 7 im Reaktor konstant blieb. Im unteren Teil des Reaktors, in der Zone 2 wurde über mehrere Gasverteiler Luft eindosiert. Das Produktgemisch wurde über ein sich an der Peripherie des oberen Teils der Zone 2 befindenden kontinuierlich zurückspülbares Absetzungssystem (Schrägklärer) vom Großteil des heterogenen Katalysator abgetrennt und über ein Filtrationssystem filtriert und mittels Gaschromatographie (GC) analysiert.

Nach 1 h Betrieb wurden jeweils 3 Proben des Produktgemisches an der 20%, 50% und 90% der Füllhöhe des Innenrohrs (Zone 1) und drei Proben an der 20%, 50% und 90% der Füllhöhe der Zone 2 genommen. Die Positionen der Messstellen sind immer von unten angegeben. Es wurde der Feststoffgehalt [in g/L] der Proben bestimmt.

Das Verhältnis C90%/C20% in der Zone 2 war kleiner als 0,1.

Die durchschnittliche Konzentration in Zone 1 <C1> wurde als Mittelwert der drei Proben aus dem Innenrohr berechnet:

$$<C1> = (C1(20\%) + C1(50\%) + C1(90\%))/3, \text{ wobei } C1(20\%) \sim C1(50\%) \sim C1(90\%) \text{ waren;}$$

waren; die durchschnittliche Konzentration in Zone 2 <C2> wurde wie folgt berechnet:

$$<C1> = (Katalysatorgesamtmasse -<C1>*V1)/V2$$

$$<C2>/<C1>\ war\ größer\ als\ 10$$

Es wurde visuell festgestellt, dass im unteren Teil der Zone 2 über Luftverteiler eingeleitete Luft sich im laufenden Betrieb fast ausschließlich in Zone 2 zu beobachten war, während Zone 1 zumindest vom im Reaktionsgemisch ungelöstem Gas frei blieb.

Sauerstoffkonzentration in der Gasphase der Zone 2 auf 20% der von unten gemessenen Füllhöhe des Reaktors war $C(O_2)20\% \sim 21$ vol%$O_2$, Sauerstoffkonzentration in der Gasphase der Zone 2 auf 90% der von unten gemessenen Füllhöhe war $C(O_2)90\% \sim 5$ vol% $O_2$, Somit $C(O_2)20\%/ C(O_2)90\% = 4{,}2$

So wurde ein kontinuierlicher störungsfreier Betrieb der Anlage für mehrere Monate gewährleistet.

Vergleichsbeispiel 1

Der Reaktor war identisch dem im Beispiel 1 verwendeten, mit Ausnahme des Innenrohrs, das fehlte. Reaktionsführung war identisch wie im Beispiel 1.

Nach 1 h wurden zwei Katalysatorsuspensionsproben, jeweils an von unten gemessenen Stellen in 20% und 90% der Füllhöhe entnommen. Es wurde ein Feststoffgehalt [in g/L] darin bestimmt. Das Verhältnis C90%/C20% war 0,31. <C1> = <C2>

Vergleichsbeispiel 2

Wie Beispiel 1 mit dem Unterschied, dass Feedlösungen nicht ins Innenrohr, sondern in die Zone 2 eingeleitet wurden.

Die Korngrößenverteilung (gemessen mit Laserbeugungsmethode) vom frischen Katalysator, vom Katalysator nach dem Test in Beispiel 1 (nach 1000 h) und nach dem Test in Vergleichsbeispielen 1 und 2 (nach jeweils 1000 h) sind in der folgenden Tabelle zusammengefasst. Aktivität und Selektivität des Katalysators nach jeweils 200 h und 1000 h ist ebenfalls für alle Beispiele angegeben:

|  | $D_{50}$ [$\mu$m] | RZA [mol MMA/kg-h] | S(MMA) [%] |
|---|---|---|---|
| Frischer Katalysator | 61,5 |  |  |
| B1 (200 h) |  | 8,5 | 95,7 |
| B1 (1000 h) | 60,4 | 8,5 | 95,6 |
| VB1 (200 h) |  | 8,5 | 95,7 |
| VB1 (1000 h) | 51,2 | 8,0 | 93,3 |
| VB2 (200 h) |  | 8,2 | 92,4 |
| VB2 (1000 h) | 60,3 | 8,0 | 90,1 |

Es wurde beobachtet, dass der mechanische Abrieb des Katalysators in einem erfindungsgemäßen Reaktor mit einem Innenrohr (Beispiel 1; B1) geringer war als der Abrieb im Reaktor ohne Innenrohr (Vergleichsbeispiel 1; VG1). Außerdem war zu beobachten, dass nach 1000 Stunden Betriebszeit die Aktivität und Selektivität des eingesetzten Katalysators abnahm.

In der Ausführungsform mit der Feedzugabe in die Zone 2 (Vergleichsbeispiel 2; VG2) war die Selektivität zu MMA wiederum deutlich geringer

Beispiel 2

Ähnlich dem Beispiel 1 aber ohne internen Filter im Reaktor nach dem Absetzungssystem. Das Reaktionsgemisch wurde abwechselnd über einen von zwei parallel installierten externen Filter mit Porosität 10 Mikrometer filtriert, wobei ein Filter ständig im Einsatz war und der andere gleichzeitig zurückgespült wurde. Der auf dem Filter zurückbleibende Katalysator wurde zurück in den Reaktor geleitet. So wurde ein kontinuierlicher störungsfreier Betrieb der Anlage für mehrere Monate gewährleistet.

Vergleichsbeispiel 3

Wie Beispiel 2 mit dem Unterschied, dass kein Absetzungssystem (kein Schrägklärer) eingesetzt wurde. Es kam kontinuierlich deutlich mehr Katalysator auf den externen Filter an. Die Taktung der Filterumstellung (Umstellung auf Rückspülmodus) musste deutlich verkürzt werden damit ein kontinuierlicher Reaktionsgemischaustrag herausgefördert werden konnte. Nach 2 Monate Betrieb musste der Betrieb gestoppt werden und die beiden eingesetzten Filter mussten intensiv mit NaOH Lösung gespült werden, bevor es mit dem Betrieb weiter gehen konnte.
Es wurde beobachtet, dass ein internes Absetzungssystem durch eine Vorabtrennung des größten Teils des Feststoffes die eingesetzten Filter deutlich entlastet und somit einen störungsfreien Betrieb fördert.

**Patentansprüche**

1. Verfahren zur Durchführung einer heterogen-katalysierten Reaktion in einem Dreiphasen-Reaktor, **dadurch gekennzeichnet, dass** sich in dem Reaktor mindestens eine flüssige, mindestens eine gasförmige und mindestens eine feste Phase befinden und der Reaktor mindestens zwei Zonen aufweist, wobei in Zone 1 das Reaktionsgemisch nach unten gefördert wird, in Zone 2 das Reaktionsgemisch nach oben gefördert wird, die Zonen 1 und 2 durch eine Trennwand voneinander getrennt sind, und dass das Verhältnis zwischen den durchschnittlichen Katalysatorkonzentrationen in Zone 2 und in Zone 1 größer als 2 ist.

2. Verfahren gemäß Anspruch 1, **dadurch gekennzeichnet, dass** das Verhältnis zwischen den durchschnittlichen Katalysatorkonzentrationen in Zone 2 und in Zone 1 größer als 5, bevorzugt größer als 10 ist.

3. Verfahren gemäß Anspruch 1 oder 2, **dadurch gekennzeichnet, dass** das für den Prozess benötigte Gas sich im laufenden Betrieb fast ausschließlich in Zone 2 befindet, während Zone 1 vom ungelösten Gas weitestgehend befreit bleibt.

4. Verfahren gemäß mindestens einem der Ansprüche 1 bis 3, **dadurch gekennzeichnet, dass** das Verhältnis zwischen der Katalysatorkonzentration in Zone 2 auf 90% der von unten gemessenen Füllhöhe des Reaktors und der Katalysatorkonzentration auf 20% der von unten gemessenen Füllhöhe kleiner 0,3, bevorzugt kleiner 0,2, noch bevorzugter kleiner 0,1 ist.

5. Verfahren gemäß mindestens einem der Ansprüche 1 bis 4, **dadurch gekennzeichnet, dass** das Verhältnis zwischen der durchschnittlichen vertikalen Strömungsgeschwindigkeit in der Zone 1 und der durchschnittlichen vertikalen Strömungsgeschwindigkeit in der Zone 2 zwischen 5 und 50, bevorzugt zwischen 10 und 40 beträgt.

6. Verfahren gemäß mindestens einem der Ansprüche 1 bis 5, **dadurch gekennzeichnet, dass** das Verhältnis des Reaktordurchmessers im oberen Teil zu dem im unteren Teil zwischen 1 und 2, bevorzugt zwischen 1,1 und 1,5 liegt.

7. Verfahren gemäß mindestens einem der Ansprüche 1 bis 6, **dadurch gekennzeichnet, dass** das Verhältnis des Durchmessers im oberen Teil der Zone 1 zu dem im unteren Teil der Zone 1 zwischen 1 und 5, bevorzugt zwischen 2 und 4 liegt.

8. Verfahren gemäß mindestens einem der Ansprüche 1 bis 7, **dadurch gekennzeichnet, dass** das Reaktionsgemisch in der Zone 1 mittels mindestens einer Pumpe oder mindestens eines Rührers nach unten gefördert wird.

9. Verfahren gemäß mindestens einem der Ansprüche 1 bis 8, **dadurch gekennzeichnet, dass** mindestens ein flüssiger Feedstrom, bevorzugt alle flüssigen Feedströme in den oberen Teil der Zone 1 eingeleitet werden.

10. Verfahren gemäß mindestens einem der Ansprüche 1 bis 9, **dadurch gekennzeichnet, dass** im oberen Teil der Zone 2 mindestens ein kontinuierlich betreibbarer und zurückspülbarer Filter installiert ist.

11. Verfahren gemäß mindestens einem der Ansprüche 1 bis 10, **dadurch gekennzeichnet, dass** Reaktionsgemisch kontinuierlich aus dem Reaktor ausgetragen und über mindestens einen externen Filter filtriert wird und der Katalysator nach der Filtration zurück in den Reaktor geleitet wird.

12. Verfahren gemäß mindestens einem der Ansprüche 1 bis 11, **dadurch gekennzeichnet, dass** Zone 2 durch Trennwände in mindestens 2 Segmente geteilt ist, und dass im unteren Teil der Zone 2 mindestens ein Gas eindosiert und fein verteilt wird.

13. Verfahren gemäß mindestens einem der Ansprüche 1 bis 12, **dadurch gekennzeichnet, dass** es sich bei der heterogen-katalysierten Reaktion um eine Oxidationsreaktion mit einem Sauerstoff-haltigen Gas handelt.

14. Verfahren gemäß mindestens einem der Ansprüche 1 bis 13, **dadurch gekennzeichnet, dass** das Verhältnis zwischen der Sauerstoffkonzentration in der Gasphase der Zone 2 auf 20% der von unten gemessenen Füllhöhe des Reaktors und der Sauerstoffkonzentration in der Gasphase der Zone 2 auf 90% der von unten gemessenen Füllhöhe größer als 2, bevorzugt größer als 4 ist.

15. Verfahren gemäß mindestens einem der Ansprüche 1 bis 14, **dadurch gekennzeichnet, dass** es sich bei der heterogen-katalysierten Reaktion um eine kontinuierliche oxidative Veresterung von Methacrolein mit Sauerstoff und Methanol zur Herstellung von Methylmethacrylat handelt.

Fig.1

**Europäisches Patentamt**
**European Patent Office**
**Office européen des brevets**

# EUROPÄISCHER RECHERCHENBERICHT

Nummer der Anmeldung

EP 16 16 6606

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 5 723 041 A (DEVANATHAN NARASIMHAN [US] ET AL) 3. März 1998 (1998-03-03) * Spalte 4, Zeile 57 - Spalte 5, Zeile 49; Abbildung 1 * ----- | 1-8 | INV. B01J8/22 B01J8/00 C07C69/54 C07C67/39 |
| X | US 2010/160460 A1 (SOTO JORGE L [US] ET AL) 24. Juni 2010 (2010-06-24) * Absätze [0025], [0028], [0031], [0032]; Abbildungen 1,5 * ----- | 1,3-7,11 | |
| X | WO 98/50493 A1 (EXXON RESEARCH ENGINEERING CO [US]) 12. November 1998 (1998-11-12) * Seite 7; Ansprüche 5,8; Abbildung 1 * ----- | 1,3,6,7,11 | |
| A | US 2009/134064 A1 (REYNOLDS BRUCE [US]) 28. Mai 2009 (2009-05-28) * Absätze [0036] - [0040]; Abbildung 1 * ----- | 1-15 | |
| A | EP 0 781 595 A1 (PRAXAIR TECHNOLOGY INC [US]) 2. Juli 1997 (1997-07-02) * Spalte 7, Zeile 51 - Spalte 8, Zeile 35; Abbildung 1 * ----- | 1-15 | RECHERCHIERTE SACHGEBIETE (IPC) |
| A | EP 1 674 449 A1 (SUMITOMO CHEMICAL CO [JP]) 28. Juni 2006 (2006-06-28) * Absatz [0022]; Abbildung 2 * ----- | 1-15 | B01J C07C |

Der vorliegende Recherchenbericht wurde für alle Patentansprüche erstellt

| Recherchenort | Abschlußdatum der Recherche | Prüfer |
|---|---|---|
| München | 10. Oktober 2016 | Veronesi, Sergio |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 16 16 6606

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

10-10-2016

| Im Recherchenbericht angeführtes Patentdokument | Datum der Veröffentlichung | Mitglied(er) der Patentfamilie | Datum der Veröffentlichung |
|---|---|---|---|
| US 5723041 A | 03-03-1998 | KEINE | |
| US 2010160460 A1 | 24-06-2010 | AU 2009330672 A1<br>CN 102292416 A<br>EP 2379675 A1<br>US 2010160460 A1<br>WO 2010074762 A1 | 21-07-2011<br>21-12-2011<br>26-10-2011<br>24-06-2010<br>01-07-2010 |
| WO 9850493 A1 | 12-11-1998 | AU 726643 B2<br>AU 7286398 A<br>BR 9809594 A<br>CA 2287456 A1<br>DE 69806753 D1<br>DE 69806753 T2<br>EP 0983328 A1<br>JP 4035171 B2<br>JP 2002501562 A<br>KR 20010012232 A<br>MY 120985 A<br>NO 995424 A<br>US 5962537 A<br>US 6270734 B1<br>WO 9850493 A1<br>ZA 9803735 B | 16-11-2000<br>27-11-1998<br>04-07-2000<br>12-11-1998<br>29-08-2002<br>21-11-2002<br>08-03-2000<br>16-01-2008<br>15-01-2002<br>15-02-2001<br>30-12-2005<br>05-11-1999<br>05-10-1999<br>07-08-2001<br>12-11-1998<br>05-11-1998 |
| US 2009134064 A1 | 28-05-2009 | KEINE | |
| EP 0781595 A1 | 02-07-1997 | BR 9606185 A<br>CA 2194033 A1<br>CN 1159437 A<br>EP 0781595 A1<br>US 5856533 A | 18-08-1998<br>29-06-1997<br>17-09-1997<br>02-07-1997<br>05-01-1999 |
| EP 1674449 A1 | 28-06-2006 | CN 1796366 A<br>EP 1674449 A1<br>KR 20060071886 A<br>SG 123723 A1 | 05-07-2006<br>28-06-2006<br>27-06-2006<br>26-07-2006 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5288673 A
- CN 104418309
- WO 2012152600 A
- US 5417930 A
- US 5969178 A
- CN 101314120
- JP 10094705 A
- JP 9248403 A
- EP 2210664 A1

**In der Beschreibung aufgeführte Nicht-Patentliteratur**

- *Journal of Fluid Mechanics,* Juni 1979, vol. 92 (03), 435-457
- Enhanced sedimentation in vessels having inclined walls. *Theory of Dispersed Multiphase Flow: Proceedings of an Advanced Seminar Conducted by the Mathematics Research Center The University of Wisconsin-Madison,* 26. Mai 1982